# EUROPEAN PATENT APPLICATION

(11) **EP 3 054 011 A1**
(43) Date of publication of application: **10.08.2016**
(21) Application number: 16152284.2
(22) Date of filing: 19.02.2013
(51) Int. Cl.: C12N 15/09, A01H 5/00, A01H 5/02, C12N 1/15, C12N 1/19, C12N 1/21, C12N 5/10, C12N 15/82, C07K 14/415

(54) **TORENIA-ORIGINATED PROMOTER CAPABLE OF ACTING IN PETALS**

(30) Priority: 24.02.2012 JP 2012038644
(62) Divisional of application: 13751778.5
(71) Applicant: Suntory Holdings Limited, Kita-ku, Osaka-shi Osaka 530-8203 (JP)
(72) Inventor: TANAKA, Yoshikazu, Osaka 618-8503 (JP); KATSUMOTO, Yukihisa, Osaka 618-8503 (JP)
(74) Representative: Keirstead, Tanis Evelyne

(57) **Abstract**

A novel promoter useful for altering the color of flowers of a plant, which is selected from the group consisting of: (1) a nucleic acid which comprises the nucleotide sequence represented by SEQ ID NO: 20, SEQ ID NO: 12 or SEQ ID NO: 21; and (2) a nucleic acid which maintains the same promoter activity as that of the nucleotide sequence represented by SEQ ID NO: 20, SEQ ID NO: 12 or SEQ ID NO: 21, and which comprises a nucleotide sequence that is produced by modifying the original nucleotide sequence by the addition, deletion and/or substitution of one or several nucleotide sequences, or can hybridize with a nucleotide acid comprising a nucleotide sequence complementary to the original nucleotide sequence under highly stringent conditions, or has a 90% or more sequence identity to the original nucleotide sequence.

SEQUENCE LISTING

<110> Suntory Holdings Limited

<120> Promoters derived from Trenia Functioning in Petals

<130> SP2012-0137(AB549)

<150> JP2012-038644
<151> 2012-02-24

<160> 21

<170> PatentIn version 3.5

<210> 1
<211> 20
<212> DNA
<213> Artificial Sequence

<220>
<223> primer T3pro

<400> 1
aattaaccct cactaaaggg
20

<210> 2
<211> 20
<212> DNA
<213> Artificial Sequence

<220>
<223> primer T7pro

<400> 2
taatacgact cactataggg
20

<210> 3
<211> 18
<212> DNA
<213> Artificial Sequence

<220>
<223> primer THF2RV

<400> 3
ctatggaaga taacaatg
18

<210> 4
<211> 5606
<212> DNA
<213> Torenia Fournieri

<220>
<221> misc_feature
<223> Trenia F3'5'H TBG10 region in pSPB3745

<400> 4
   aattaaccct cactaaaggg aacgaattcg gatcctcatg ttatgaaata taaatatgaa
   60
   tgggattaaa cttgtagtta attggtatca ttaaaaaaaa aagaaaaaag aaattgaagt
   120
   aaaataatcg caggtaactt gaagaaaata ttaataacta aaataataat aataataata
   180
   ataataataa gcaggtttga aacacacacc ttgggaattt gttgttcttg acagcctttt
   240
   gaccatattt cctccatcga tatccatcgt caagtatatc gacttggctt cttgtctgaa
   300
   aggcgtattt atgcttctta ttcttcttct tcttattctt ctcctcgctc tttattgctt
   360
   ccttattatt atcagacttg tttgggtcct cctctgcgag agttattgca gtactattat
   420
   tattaaagct actactggcg attgtaatag catctgacat gcttttaata ttcatctcca
   480
   tgctgttata gccaaaacta ctactgctag gcctgtgatc ttgaaacaga tcataatgat
   540
   cttgtggaaa tgaagaagaa gactaaaaca ttattggata gttattattg tccatgagaa
   600
   gagaagatga taatagataa caaataaatg atggttttcc ttgttttttt ttataataac
   660
   tatgaatgtg aagaatagtg gattaagaaa agtacaagtc gtcgtgtggt gttgtttaga
   720
   atgagagaaa agtttattat acaacaagta ccgtcccagc tcgtttaaag atgaaaggcg
   780
   gcggcttgac ttgagggcga atattattat ttatttaaat aagttacatc ataaacatat
   840
   tttgtgcata gtgaatgtta aaccaaaagc gaaatggcta tgatcgcagt attaatatat
   900
   tatctgagga gataaatttg atcgaattga ttagcaggga taataattat agagatgatc
   960
   caacttgctt gatgaaatta tggatctata ctcaatctta aaacgaattt acttacttac
   1020
   atagcgtgaa atagctaatt ggaaatgaaa agaaaaagaa aaagaaaaat tgttagaaat
   1080
   atgacttgga gggagggttt tgaatcttca aacatacaca aatttaatta atacattacg
   1140
   gtcgttaagc aaatcatttt tattctacgg acagtagata ttgcaataat agttagaaaa
   1200
   gttggtaaag aattagtaca gttggtcaaa cactattatt attattatta ttattgttat
   1260
   tattattatt attctataaa tctgataata aaaacaaata gcatatgaat aatagcatat
   1320
   aaatgctgga tccgatttaa ttttaatttt agttaaatag caactttcag taaatttttt
   1380
   tagttaactg cctctggaaa aatgaaatgc gtgggtggga gcggttcgga tttggtacgg
   1440
   aaaatgcttt tctccgcatt gtgcggataa atctggaccg tgcgatcagg cccaaaatta
   1500
   atccagtggt gaaggacgag agggggtaaa tctggaattt ttttttttac tcataataaa
   1560
   aaacgattat atcttttgaa tgccaaaaca aaataaaaaa ataaaaacac caaatttcat
   1620
   catttttaat tccctacaaa aagagatcca tcttgctata taaattttat atttttaggg
   1680
   tttagggttt agggtttacg gcctggcaca gacacatcta aaacgaagca caactcagct
   1740
   aaacgaagca caaattcatt attatgaagc acatattcat tagtatgaag cacatattca
   1800
   tttttatgaa gcgcaattca tttgtatgaa gcacatattc atttttatga agcgcaattc
   1860
   atttgtatga agcacaaaat catttgtatg aaacttataa tcatttgtat gaagcacaaa
   1920
   ttcatttata tgaagcaaaa attcatttgt atgaacgcaa ttcatttgta tgaatcacaa
   1980
   attaatttgt atgaagcaca aattaatttg tatgaagcac aactcatttg tacaaaatca
   2040
   tacaaataat tataagtacc atctaattga agcacaaatt catttgtttg tatgaagcac
   2100
   aaattcattt gtataaagca caaattcatt tgtacaatac atccaaataa tatataaagc
   2160
   accatctaaa cgaagtacaa attcatttgt atgaagcaca aattcgtaca aaaaatattt
   2220
   aaagcacaat ctaaaacgaa gcacagattc atttgtatga agcacaaatt catttgtaca
   2280
   atacatccaa ataatatata aagcaccatc taaacgaagc acaaattcat ttgtatgaag
   2340
   cacaaattca tttgtacaaa ttcatacaaa aaatatttaa agcacaatct aaaacgaagc
   2400
   acagattcat ttgtatgaag cacagattca tcaggggtat tttagacaaa aacccgacga
   2460
   atatcatccg ttagatttat tttgagtaaa tccaatggcc cattactttt gtccgcacat
   2520
   acggagaaaa gtaccgtccg catttgaacc agaggggatg ggagggactg cgaacataat
   2580
   ataattcggg tcgatggcgt accaaaaaat ggtcaaataa ataatgctac aagttaaatg
   2640
   cacgtgattc tcttgttgac cgttaaatgt tgatttctat tttgatcgaa ttttatagat
   2700
   attagctcag ctgctgagac tgagagagag attattttag aaacggatct gatcaagatc
   2760
   gctcgtgatt attctcatca tctcgtgtag catttagatt tggtaaaacc gtaaaaatat
   2820
   cttgtcttaa cttcttccta ttaattccac aatctcacgc aaaaatgtat ttttacagcg
   2880
   acatgccttg aggaggaatt tcagtgctaa agacaaaata gacttagaaa tgtgccgttt
   2940
   gattaatttt ggtagttaaa aaaaaaaaat tatttattta tttatgctta gccataataa
   3000
   ttaggacgga aacaagaaga atttgcccat ttttaaggcc tacgaatagt atacgggcca
   3060
   taactttatg gggacatttt tcaggcctac ccaatatgat tgaatgtgcc tccactccaa
   3120
   ttatcaaaaa gaaacgccga ctgagcttga gcacgatttt caaatgtcta ctccacagtg
   3180
   caaaaattgc acgatcaatt ctgatgaaat gtattcccat ctatgtagat gaatgggagc
   3240
   gtttgggatc caacactcgt tttttctacc tatttcgacg gttttctctg gtcaggtggt
   3300
   ttccgagagg ggactgaaat atacagtaca gttgttgaga tattgatgag atcaatcaag
   3360
   tcgtgtaagc ttatctttct gaaaatataa ttatatttaa ttgattatcc taccgcttgt
   3420
   caattttttc tattgcgagt gtgttttggt gtaaagagta gctatcgatg ctgaagagat
   3480
   ttttatcaag taagatgatg tggagaacta actgattgtt tgttttgttg ccagcgatat
   3540
   attgatgatg atgttctttg caattagagt tattctactt aacaaacttt ttctcttgtc
   3600
   ataacataca catagatact gcatgctttt ccatgattca ttctatctaa taaaggtcaa
   3660
   tctactcccc actttcatcc atgccaatga aacacataca taaatggaaa catatcataa
   3720
   tttaggtgtg gccctattag ttttcttctc gttgaacgtc ggttgatggc ctaaaaaaac
   3780
   gtgacaaacc acacagttgt cacccaacgt actccattga tacacatata aagctcagtg
   3840
   attgattatt cataaacact caactggttg agcactattt gcctcttctt tgctagattc
   3900
   acatcgagta agtactccgt ccgacctttt attgtatggt ataatcgtaa gaaaacaaat
   3960
   taagcagcta gtcaccattc agtatgacag tagctactac acttgtcgtt gtgttgagta
   4020
   gagagttgtt gtatgtgttg gttgtctaca ttctggtctc aaaatcacta tctaccatca
   4080
   tcgtcgtctc ccgcaaacgg ttaccccccg gcccaacggg cttccccgtg gtcggtgccc
   4140
   tcccattgtt gggcagcatg cctcacgtgg cgttggccaa aatggcaaaa aaatacgggc
   4200
   ccgtcatgta ccttaaaacg ggcacactgg gcatggtggt ggcctccacg ccttcctcgg
   4260
   caagagcgtt tctcaagaca ctggactcca acttctcgaa ccggcccgcc aatgccggtg
   4320
   ccacccacct ggcctacggt gcgcaggaca tggtgttcgc tccctacggc cccagatgga
   4380
   gactgctgag aaggttaagc aacctccaca tgctcggcgg caaggccctc gacggatggg
   4440
   caaacgtcag ggcctcggag ttggggcaca tgctggaggc catgaaccgg gcgagccggg
   4500
   agggggaggt ggtggtggtg ccggagatgc tggtgtacgc catggccaac atgatcgggc
   4560
   aagtcacact gagccggcgg gtcttcgtga ccaaagggga ggaagtgaac gagtttaagg
   4620
   acatggtggt ggagctcatg acgtctgctg gatacttcaa catcggggac tttgtcccga
   4680
   tccttgcttg gatggacttg caagggattg agagagggat gaaagctctt cacaaaaagt
   4740
   ttgacaagct catcacgaga atgttggagc agcatgaggc gtcggcccac aagcccgatt
   4800
   ttctcgacgc catcatcgcg gccagccttg acgattattc gagtgaggag gagaggctca
   4860
   gtacgtccaa catcaaagct ctcttattgg taaggctaag ctttattaat tttacccatc
   4920
   attgaatatg catgcaacaa caagaggtcc tatcattgaa tatgcatgtg tgtgtgtaga
   4980
   acttgtttac tgctggaacg gacacatcat cgagcacgat agaatgggca ctctcggaga
   5040
   tgctgaaaaa ggggaaaatc ctaaagaggg ctcaagagga gatggacaga gtggtgggac
   5100
   gagagaggcg cttggtggaa tcggacatag agaaactggg gtacctgaaa gccatatgca
   5160
   aagagacgtt ccggaagcat ccttcgacgc cgctgaacct gcctcggatc tcgtcggagg
   5220
   catgcgtggt gaacggccac tacataccaa agggcacgag gctgagcgtc aacatatggg
   5280
   ccatcgggag agaccctgag gtgtgggagg atccgttgga gtttaacccg gacaggtttc
   5340
   tgcactcgaa gatggatccg agggggaacg atttcgagct catgcccttc ggtgcgggtc
   5400
   gaaggatatg tgccgggacg aggatgggga tagtcctcgt cgagtatata ttggccactt
   5460
   tggtgcattc tttcggctgg aaagcggctg atcaagataa caacatcatg aatatggagg
   5520
   aggcctttgg gattgctctg cagaaggcca cacctcttaa ggctctcgtt actcctcgcc
   5580
   tgcctttaca ttgttatctt ccatag
   5606

<210> 5
<211> 21
<212> DNA
<213> Artificial Sequence

<220>
<223> primer TBG10proFW

<400> 5
tgaaatataa atatgaatgg g
21

<210> 6
<211> 20
<212> DNA
<213> Artificial Sequence

<220>
<223> primer TBG10proRV

<400> 6
actgaatggt gactagctgc
20

<210> 7
<211> 3942
<212> DNA
<213> Torenia Fournieri

<220>
<221> misc_feature
<223> Trenia F3'5'H TBG10 in pSPB3764

<220>
<221> primer_bind
<222> (1)..(21)
<223> primer TBG10proFW binding

<220>
<221> primer_bind
<222> (3923)..(3942)
<223> primer TBGproRV binding

<400> 7
   tgaaatataa atatgaatgg gattaaactt gtagttaatt ggtatcatta aaaaaaaaag
   60
   aaaaaagaaa ttgaagtaaa ataatcgtag gtaacttgaa gaaaataata ataactaaaa
   120
   taataataat aataataata ataataagca ggtttgaaac acacaccttg ggaatttatt
   180
   gttcttgaca gccttttgac catatttcct ccatcgatat ccatcgtcaa gtatatcgac
   240
   ttggcttctt gtctgaaagg cgtatttatg cttcttattc ttcttcttct tattcttctc
   300
   ctcgctcttt attgcttcct tattattatc agacttgttt gggtcctcct ctgcgagagt
   360
   tattgcagta ctattattat taaagctact actggcgatt gtaatagcat ctgacatgct
   420
   tttaatattc atctccatgc tgttatagcc aaaactacta ctgctaggct tgtgatcttg
   480
   aaacagatca taatgatctt gtggaaatga agaagaagac taaaacatta ttggatagtt
   540
   attattgtcc atgagaagag aagatgataa tagataacaa ataaatgatg gttttccttg
   600
   tttttttttt ataataacta tgaatgtgaa gaatagtgga ttaagaaaag tacaagtcgt
   660
   cgtgtggtgt tgtttagaat gagagaaaag tttattatac aacaagtacc gtcccagctc
   720
   gtttaaagat gaaaggcggc ggcttgactt gagggcgaat attattattt atttaaataa
   780
   gttacatcat aaacatattt ttgtgcatag tgaatgttaa accaaaagcg aaatggctat
   840
   gatcgcagta ttaatatatt atctgaggag ataaatttga tcgaattgat tagcagggat
   900
   aataattata gagatgatcc aacttgcttg atgaaattat ggatctatac tcaatcttaa
   960
   aacgaattta cttacttaca tagcgtgaaa tagctaattg gaaatgaaaa gaaaaagaaa
   1020
   aagaaaaatt gttagaaata tgacttggag ggagggtttt gaatcttcaa acatacacaa
   1080
   atttaattaa tacattacgg tcgttaagca aatcattttt attctacgga cagtagatat
   1140
   tgcaataata gttagaaaag ttggtaaaga attagtacag ttggtcaaac actattatta
   1200
   ttattattat tattgttatt attattatta ttctataaat ctgataataa aaacaaatag
   1260
   catatgaata atagcatata aatgctggat ccgatttaat tttaatttta gttaaatagc
   1320
   aactttcagt aaattttttt agttaactgc ctctggaaaa atgaaatgcg tgggtgggag
   1380
   cggttcggat ttggtacgga aaatgctttt ctccgcattg tgcggataaa tctggaccgt
   1440
   gcgatcaggc ccaaaattaa tccagtggtg aaggacgaga gggggtaaat ctggaatttt
   1500
   ttttttactc ataataaaaa acgattatat cttttgaatg ccaaaacaaa ataaaaaaat
   1560
   aaaaacacca aatttcatca tttttaattc cctacaaaaa gagatccatc ttgctatata
   1620
   aattttatat ttttagggtt tagggtttag ggtttacggc ctggcacaga cacatctaaa
   1680
   acgaagcaca actcagctaa acgaagcaca aattcattat tatgaagcac atattcatta
   1740
   gtatgaagca catattcatt tttatgaagc gcaattcatt tgtatgaagc acatattcat
   1800
   ttttatgaag cgcaattcat ttgtatgaag cacaaaatca tttgtatgaa acttataatc
   1860
   atttgtatga agcacaaatt catttatatg aagcaaaaat tcatttgtat gaacgcaatt
   1920
   catttgtatg aatcacaaat taatttgtat gaagcacaaa ttaatttgta tgaagcacaa
   1980
   ctcatttgta caaaatcata caaataatta taagtaccat ctaattgaag cacaaattca
   2040
   tttgtttgta tgaagcacaa attcatttgt ataaagcaca aattcatttg tacaatacat
   2100
   ccaaataata tataaagcac catctaaacg aagtacaaat tcatttgtat gaagcacaaa
   2160
   ttcgtacaaa aaatatttaa agcacaatct aaaacgaagc acagattcat ttgtatgaag
   2220
   cacaaattca tttgtacaat acatccaaat aatatataaa gcaccatcta aacgaagcac
   2280
   aaattcattt gtatgaagca caaattcatt tgtacaaatt catacaaaaa atatttaaag
   2340
   cacaatctaa aacgaagcac agattcattt gtatgaagca cagattcatc aggggtattt
   2400
   tagacaaaaa cccgacgaat atcatccgtt agatttattt tgagcaaatc caatggccca
   2460
   ttacttttgt ccgcacatac ggagaaaagt accgtccgca tttgaaccag aggggatggg
   2520
   agggactgcg aacataatat aattcgggtc aatggcgtac caaaaaatgg tcaaataaat
   2580
   aatgctacaa gttaaatgca cgtgattctc ttgttgaccg ttaaatgttg atttctattt
   2640
   tgatcgaatt ttatagatat tagctcagct gctgagactg agagagagat tattttagaa
   2700
   acggatctga tcaagatcgc tcgtgattat tctcatcatc tcgtgtagca tttagatttg
   2760
   gtaaaaccgt aaaaatatct tgtcttaact tcttcctatt aattccacaa tctcacgcaa
   2820
   aaatgtattt ttacagcgac atgccttgag gaggaatttc agtgctaaag acaaaataga
   2880
   cttagaaatg tgccgtttga ttaattttgg tagttaaaaa aaaaaaaatt atttatttat
   2940
   ttatgcttag ccataataat taggacggaa acaagaagaa tttgcccatt tttaaggcct
   3000
   acgaatagta tacgggccat aactttatgg gggacatttt caggcctacc caatatgatt
   3060
   gaatgtgcct ccactccaat tatcaaaaag aaacgccgac tgagcttgag cacgattttc
   3120
   aaatgtctac tccacagtgc aaaaattgca cgatcaattc tgatgaaatg tattcccatc
   3180
   tatgtagatg aatgggagcg tttgggatcc aacactcgtt ttttctacct atttcgacgg
   3240
   ttttctctgg tcaggtggtt tccgagaggg gactgaaata tacagtacag ttgttgagat
   3300
   attgatgaga tcaatcaagt cgtgtaagct tatctttctg aaaatataat tatatttaat
   3360
   tgattatcct accgcttgtc aatttttctt attgcgagtg tgttttggtg taaagagtag
   3420
   ctatcgatgc tgaagagatt tttatcaagt aagatgatgt ggagaactaa ctgattgttt
   3480
   gttttgttgc cagcgatata ttgatgatga tgttctttgc aattagagtt attctactta
   3540
   acaaactttt tctcttgtca taacatacac atagatactg catgcttttc catgattcat
   3600
   tctatctaat aaaggtcaat ctactcccca ctttcatcca tgccaatgaa acacatacat
   3660
   aaatggaaac atatcataat ttaggtgtgg ccctattagt tttcttctcg ttgaacgtcg
   3720
   gttgatggcc taaaaaaacg tgacaaacca cacagttgtc acccaacgta ctccattgat
   3780
   acacatataa agctcagtga ttgattattc ataaacactc aactggttga gcactatttg
   3840
   cctcttcttt gctagattca catcgagtaa gtactccgtc cgacctttta ttgtatggta
   3900
   taatcgtaag aaaacaaatt aagcagctag tcaccattca gt
   3942

<210> 8
<211> 22
<212> DNA
<213> Artificial Sequence

<220>
<223> primer TBG10proXbaIRV

<400> 8
tctagactga atggtgacta gc
22

<210> 9
<211> 2307
<212> DNA
<213> Torenia Fournieri

<220>
<221> misc_feature
<223> Trenia F3'5'H TBG16 region in pSPB3746

<400> 9
aattaaccct cactaaaggg aacgaattcg gatcctattg cactcgtttt ttctcgacga
60

ttttctctgg tcaggtccgt tgtttggatt ccgacaggag actgaaatat acagttgttg
120

agctgctgat gagatcaatc aagtcatcta agcttatctt tctgaaatat ataattatat
180

ttaattgatt atcgtaccgc ttgtcaattt tttgtctatt gcgagtgcgt tctggtggaa
240

ttggatgtgg ccgcgatgcc tggtgtagtg tagctgaaga gattttcgag tgattgctgg
300

ttttgttgcc agcgatattg atgttctttg cagttaagag ttattctact taccaatttt
360

tctcataacg tacacaaaaa accattctat ctatctaata aaggtcaatc tactcaccac
420

tttcatccat gccataaatg gaaacatatc ataagttagg tgtgtatcgt tggataattt
480

tgccctatta cttttttttt tcctcgttga acgtcggttg atggccttaa aaaacgtgac
540

aaaccacaca gttgtcaccc aactccattg atacacatat aaagctcagt aattgattat
600

ctcaactagc tgagcattat ttgccctttc tttgctagat tcacattgtg taagtactcg
660

gaccttttgt tgtataatca gaagaaaaca aattaagcgg ctagtcacca ttcagtatga
720

cagtagctac tacacttgtc gttgtgttga gtagagagtt gttgtatgtg ttggttgtct
780

acgttctggt gcggtcaata ctcatctcaa agtcactgtc taccatcatc gtctcccgca
840

aacggttacc ccccggccca acgggcttcc ccgtggtcgg tgccctccca ttgttgggca
900

acatgcctca cgtggcgttg gccagaatgg caaaaaaata cgggcccgtc atgtacctta
960

aaacgggcac actggggatg gtggtggcct ccacgccttc gtcggcaaaa gcgttcctca
1020

agacactgga ctccaacttc tcgaaccgcc ctggcaatgc cggtgccacc cacctggcct
1080

acggggcgca ggacatggtg tttgctccct acggccccag atggagactg ctgagaaggt
1140

taagcaatct ccacatgctc ggaggcaagg ccgtggacgg atgggcaaac gtcagggcct
1200

tgagttgggg cacatgctgg aggccatgaa ccgggcgagc cgcgacgggg aggtggtggt
1260

ggtgccggag atgctggtgt acgccatggc caacatgatc gggcaagtga tactgagccg
1320

gcgggttttc gtcaccaaag ggatgaagt gaacgagttt aaggacatgg tggtggagct
1380

catgacgact gctggatact tcaacatggg cgactttgtc ccgatccttg cttggatgga
1440

cttgcaaggg attgagagag ggatgaaagc ccttcacaaa aagttggaca agctcatcac
1500

gagaatgttg gagcagcatg cggcgtcggc ccacacgccc gattttctcg acgccatcag
1560

ccttgacgac tattcgagtg ggagaggct cagtacgacc aacatcaaag ctctcttatt
1620

ggtaagcttt atatattttc cccatgccat gccatccatc ctattgaaat atgtgtataa
1680

gtggtgtaga acttgtttac tgcgggaacg gacacatcat cgagcacgat agaatgggcg
1740

ctctcggaga tgctgaaaaa ccggaaaatc ctaaggagga aatggacaga gtggtgggac
1800

gagagaggcg gctggtggaa tcggacatag aaaaactgcc gtacctggta gccatatgca
1860

aggagacttt ccggaagcat ccttcgacgc cgctgaaccg gcctcggatg tcgtcggagg
1920

cgtgcgtggt gaacggtcac tacataccca agggcacgag gctgagcgtc aacatatggg
1980

ccatcgggag agaccctgag gtgtgggagg atccgttgga atttaacccg gacaggtttc
2040

tgcactcgaa gatggatccg agggggaacg atttcgagct catgcccttc ggtgcgggtc
2100

gaaggatatg tgccgggacg aggatgggga ttgtcctcgt cgagtacata ttgggcactt
2160

tggtgcattc tttccaatgg aagctgccgc ctcatcaaga caccatcaat atggaggagg
2220

cctttgggat tgctctgcag aaggccatac ataccacttg aggctctcgt tactcctcgc
2280

cttcctttac attgttatct tccatag
2307

<210> 10
<211> 21
<212> DNA
<213> Artificial Sequence

<220>
<223> primer TBG16proFW

<400> 10
tcctattgca ctcgtttttt c
21

<210> 11
<211> 20
<212> DNA
<213> Artificial Sequence

<220>
<223> primer TBG16proRV

<400> 11
actgaatggt gactagccgc
20

<210> 12
<211> 685
<212> DNA
<213> Torenia Fournieri

<220>
<221> misc_feature
<223> Trenia 3'5'H TBG16 region in pSPB3758

<220>
<221> primer_bind
<222> (1)..(21)
<223> primer TBG16proFW binding

<220>
<221> primer_bind
<222> (666)..(685)
<223> primer TBG16proRV binding

<400> 12
tcctattgca ctcgtttttt ctcgacgatt ttctctggtc aggtccgttg tttggattcc
60

gacaggagac tgaaatatac agttgttgag ctgctgatga gatcaatcaa gtcatctaag
120

cttatctttc tgaaatatat aattatattt aattgattat cgtaccgctt gtcaattttt
180

tgtctattgc gagtgcgttc tggtggaatt ggatgtggcc gcgatgcctg gtgtagtgta
240

gctgaagaga ttttcgagtg attgctggtt ttgttgccag cgatattgat gttctttgca
300

gttaagagtt attctactta ccaatttttc tcataacgta cacaaaaaac cattctatct
360

atctaataaa ggtcaatcta ctcaccactt tcatccatgc cataaatgga aacatatcat
420

aagttaggtg tgtatcgttg gataattttg ccctattact tttttttttt cctcgttgaa
480

cgtcggttga tggccttaaa aaacgtgaca aaccacacag ttgtcaccca actccattga
540

tacacatata aagctcagta attgattatc tcaactagct gagcattatt tgccccttct
600

ttgctagatt cacattgtgt aagtactcgg accttttgtt gtataatcag aagaaaacaa
660

attaagcggc tagtcaccat tcagt
685

<210> 13
<211> 24
<212> DNA
<213> Artificial Sequence

<220>
<223> primer TBG16proBamHI

<400> 13
ggatccactg aatggtgact agcc
24

<210> 14
<211> 21
<212> DNA
<213> Artificial Sequence

<220>
<223> primer TFNSR3

<400> 14
attcctaatg ggctgaaagt g
21

<210> 15
<211> 4173
<212> DNA
<213> Torenia Fournieri

<220>
<221> misc_feature
<223> Trenia FNS transcriptional region in pSPB3747

<400> 15
tccttggcaa ttttattttt attttgttta atgaagaagc gaagtacagt tccttggatc
60

ctgcaaatga aaccccatca gtgtccacaa atcatcatat atcttgtcct tcaatttcta
120

taccctacaa aggttagtcc ttcagtcttt ctcaagcaaa agtcattttc caatggctta

180

gaaagaaaaa gacataactt ttgtatgtca tcaatacatt agctactgta ggcattttga
240

cctcttcaat attagaagga aaataaaaaa agaaacaaag cacagagacc attaaagcta
300

cgtacatctc cagcttagct tggcaataat atttctaaaa taatttcttt ctccccatat
360

ttcatcgtag cagaggcctt aatttcaaga attcttacaa gggtttctac ttattgtaat
420

atcatagggt ttatttggta tttctttcct tcaagctgtt tcacatcaac atattcaaga
480

tgtttcccaa aaacacatac ctgattcctc agatttctcc tattcattct cgcgccacta
540

cttcttcagt tcttgacctg aacgaccccg agatcttgta ttgtcaccac caccaagaca
600

tgttctctgc tcacaattat atatcagctt caaataatgc tcaggctctc gagccttcga
660

tgatgttcaa tcaagatatc tcaggcctaa acattggaga tccgaagatg tcgcccgtta
720

aacactctga atgaactctc actagaacat ccaatccatt cccgatatct ccctgcacaa
780

atacgtacac gaaaggttag tcgagataca gcatagcatt tttgaggttg aattattatt
840

tttaccttca cgagagctat atgttccatc tcgtcgatct tcgatcggta gctatgagcc
900

tgaaacaccc cccatttcgt tggcagacga gtaattgctg ttctctccac cagcttttcc
960

cttttcctca tgtacctgcg gatagatact tgaataatat tatcgttgtc actcgttctt
1020

tgcagtctct atatgttgtg tcgaggcttt gagctttacc taatgagatc tgtgatcgaa
1080

acgataggga tactatgatc cgagcccaat tttctcaaat gggccatcga ggccatagaa

1140

ccatcttctc tatcgacgat agtcgagagg acagagacag gtctcaaacc agcttgcatc
1200

accaagtcca cagaggcctc ggtgtgacca gcccgacgaa gaacacctcc gtttctatac
1260

ttgagcggga acacgtggcc cggcttgttg aaatcttcgg gcttcgactc gagagaagac
1320

agagcaagta cagttttagc cctgtccgaa gctgacacgc cagtagatgt ttcagcttta
1380

gcgtcctaaa ttgtttaaag agattattgg tgagacaaaa gaaaaggtta atgctttcgg
1440

gacatgacac atctaagatc gatctacacg ggcaataagc gtagacaacg ggtaatatga
1500

ttaccactgt gatagtgaac gaaggagcag aagaatcatg ttctttctcg ggattcatca
1560

atggcagctt caatctctcg aggtcgtctc ctttcatccc gacagagacg atgcccgatc
1620

cgtgtttgat cataaaggcg atggccttct cgctcgcgtg agaagctgcc attatgagat
1680

ttccttctag gctatcgccg ctctcatcat ctacgacaat cacaaacttc aaacacgaaa
1740

aggaaatttc agagacagac gctatgggtt ttgaaattgc gtcgcaaaat caaaacagag
1800

cctgtgttag cagtaccttt ccctgtcgca gagcagatag agcattttca atggatgagt
1860

aaccctgaga tggacgatca ggatcgcctt cggcgtcact cacgaagaaa tcaacggttt
1920

cgtgagtgat ctctgcttcg agcgtttcaa agggtgtagc gggagatgac gactcgtcat
1980

caaataggga gccatttaag gttgcccgtt tcttgattga actctcatcg tcgtcagcat
2040

gagataagtc tcctgctgaa aatttaagag catgacagta gtaagtgttg agcttgcatg

2100

gtctgcttct gatgattccg aatcccaggt tggaatgcga agattttgac gttataaaca
2160

atagcgattg ctgaagacag ataatgcaat ccattgattc ttgaattgtt ttatttctat
2220

ttggatagta aatgaagagc tttgggttat atccagtaga tgcagagatt tggaaccaca
2280

tgaatgtagg aatagagaca acaaggccaa gaatcccaac tagtagataa tatcgtgaat
2340

gatatttgta aagaacgttg tattaaattc aggacactta attgatgaat cacaatcaaa
2400

taatattatt ttgcagtatc tggtacataa gtatgatata aatgataagt gccataactg
2460

tgagaatata aactataatc gtaataaaaa tttgtatagc ataaaatatt taatatttac
2520

tataaattca ataaaatcaa tgctacatga ttatgtaaga tgatgtggat tttcttaaca
2580

aaatgatttt ggcttgatcg tgatgatact attaaaaaat aatgaagttt gtctaggaca
2640

tggcattggt ttatagatag atgtcctcat acgtttattg gcacttgaca tttgtttctt
2700

tttattcttg tgaattggga tatgtggatt tataaatatt tttagaagtc tggcttgtat
2760

ttctgatact aatttttatg atataatttc tttatgaatt aattgatatt aaaggctcat
2820

ttgaagtttg cggatgattc aatgcgagca atagcgtata tattgtgatt ggttggtgca
2880

ctgcacatat aggctcgcat cgaatcatcc gccgtttgct taggaaatta taagactcat
2940

tttattaatt taaaaataga atttgggtga aaggtagacg tacggtacgg ctacggtacc
3000

taaccttcga ggcttagact aagggtaaag aaaagaccag gaaaagaaaa aatctagaat

3060

cgttaaaata ttaaatgcaa ttaaatgaaa ataaaaaagg ataaataaag atccacacca
3120

cacctatcaa ttgtggagtc taattgtttg aacttgatac aatacaatga tatctgaagt
3180

atcgtatcga aatacacacg ttatgttttt cctatttggc acatcatatt tattcttggt
3240

ttttcaaatc caagatacaa aaactcataa aatacctcag tcaccaaaaa taacactaac
3300

agatttcaaa catactgcat gattatttaa aattaaaacc gagaagtgag atgtccaaga
3360

attgtttgtt aaaaccctca aaactctaag gatattacct aataagtcgt tggatgtaat
3420

tcttatgatg atcacaaatc acacattcaa tagttcaacg caattaccga aagtagcagg
3480

gcgaaaatgt cattatcatg atcaaaggga ccatgaatgt aattgcgcaa tcagacgacg
3540

tgtcggtgaa accaaaaaat ttagaagaaa atatagctca ctcaccacca ctactcgaac
3600

ctaacaccgc attaaatatc ctacaccact tcctttctat aaagaacaca tcaacccaca
3660

aagcttttat ctcgtttaca tcgaaaccgc tatatcatta catttacaac agcgctaaaa
3720

aaatatatat aaagcatgga cacagtctta atcacactct acaccgccct gttcgtcatc
3780

accaccacct tcctcctcct cctccgccga aggggaccac cgtctccgcc cggtcctctc
3840

tccctaccca taattggcca cctccacctc ctcggcccaa gactccacca cacgttccat
3900

gaattctcac tcaaatacgg cccattgatc cagctcaagc tcggctcgat cccgtgcgtc
3960

gtggcctcga cgcccgagct cgcgagagag tttcttaaga cgaacgagct cgcgttctcc

4020

tctcgcaagc actctacggc catagacatc gtcacctacg actcgtcctt tgctttctct
4080

ccgtacggac cctactggaa gtacatcaag aaactgtgta cctacgagct gctcggagcg
4140

aggaacctcg gacactttca gcccattagg aat
4173

<210> 16
<211> 22
<212> DNA
<213> Artificial Sequence

<220>
<223> primer TFNS1proFW

<400> 16
caaatgaaac cccatcagtg tc
22

<210> 17
<211> 24
<212> DNA
<213> Artificial Sequence

<220>
<223> primer TFNS1proRV

<400> 17
gctttatata tattttttta gcgc
24

<210> 18
<211> 3735
<212> DNA
<213> Torenia Fournieri

<220>
<221> misc_feature
<223> FNS transcriptional region in pSPB3759

<220>
<221> primer_bind
<222> (64)..(85)
<223> TFNS1proFW binding

<220>
<221> primer_bind
<222> (3712)..(3735)
<223> TFNS1proRV binding

<400> 18
tccttggcaa ttttattttt attttgttta atgaagaagc gaagtacagt tccttggatc
60

ctgcaaatga aaccccatca gtgtccacaa atcatcatat atcttgtcct tcaatttcta
120

taccctacaa aggttagtcc ttcagtcttt ctcaagcaaa agtcattttc caatggctta
180

gaaagaaaaa gacataactt ttgtatgtca tcaatacatt agctactgta ggcattttga
240

cctcttcaat attagaagga aaataaaaaa agaaacaaag cacagagacc attaaagcta
300

cgtacatctc cagcttagct tggcaataat atttctaaaa taatttcttt ctccccatat
360

ttcatcgtag cagaggcctt aatttcaaga attcttacaa gggtttctac ttattgtaat
420

atcatagggt ttatttggta tttctttcct tcaagctgtt tcacatcaac atattcaaga
480

tgtttcccaa aaacacatac ctgattcctc agatttctcc tattcattct cgcgccacta
540

cttcttcagt tcttgacctg aacgaccccg agatcttgta ttgtcaccac caccaagaca
600

tgttctctgc tcacaattat atatcagctt caaataatgc tcaggctctc gagccttcga
660

tgatgttcaa tcaagatatc tcaggcctaa acattggaga tccgaagatg tcgcccgtta
720

aacactctga atgaactctc actagaacat ccaatccatt cccgatatct ccctgcacaa

780

atacgtacac gaaaggttag tcgagataca gcatagcatt tttgaggttg aattattatt
840

tttaccttca cgagagctat atgttccatc tcgtcgatct tcgatcggta gctatgagcc
900

tgaaacaccc cccatttcgt tggcagacga gtaattgctg ttctctccac cagcttttcc
960

cttttcctca tgtacctgcg gatagatact tgaataatat tatcgttgtc actcgttctt
1020

tgcagtctct atatgttgtg tcgaggcttt gagctttacc taatgagatc tgtgatcgaa
1080

acgataggga tactatgatc cgagcccaat tttctcaaat gggccatcga ggccatagaa
1140

ccatcttctc tatcgacgat agtcgagagg acagagacag gtctcaaacc agcttgcatc
1200

accaagtcca cagaggcctc ggtgtgacca gcccgacgaa gaacacctcc gtttctatac
1260

ttgagcggga acacgtggcc cggcttgttg aaatcttcgg gcttcgactc gagagaagac
1320

agagcaagta cagttttagc cctgtccgaa gctgacacgc cagtagatgt ttcagcttta
1380

gcgtcctaaa ttgtttaaag agattattgg tgagacaaaa gaaaaggtta atgctttcgg
1440

gacatgacac atctaagatc gatctacacg ggcaataagc gtagacaacg ggtaatatga
1500

ttaccactgt gatagtgaac gaaggagcag aagaatcatg ttctttctcg ggattcatca
1560

atggcagctt caatctctcg aggtcgtctc ctttcatccc gacagagacg atgcccgatc
1620

cgtgtttgat cataaaggcg atggccttct cgctcgcgtg agaagctgcc attatgagat
1680

ttccttctag gctatcgccg ctctcatcat ctacgacaat cacaaacttc aaacacgaaa

1740
aggaaatttc agagacagac gctatgggtt ttgaaattgc gtcgcaaaat caaaacagag
1800
cctgtgttag cagtaccttt ccctgtcgca gagcagatag agcattttca atggatgagt
1860
aaccctgaga tggacgatca ggatcgcctt cggcgtcact cacgaagaaa tcaacggttt
1920
cgtgagtgat ctctgcttcg agcgtttcaa agggtgtagc gggagatgac gactcgtcat
1980
caaataggga gccatttaag gttgcccgtt tcttgattga actctcatcg tcgtcagcat
2040
gagataagtc tcctgctgaa aatttaagag catgacagta gtaagtgttg agcttgcatg
2100
gtctgcttct gatgattccg aatcccaggt tggaatgcga agattttgac gttataaaca
2160
atagcgattg ctgaagacag ataatgcaat ccattgattc ttgaattgtt ttatttctat
2220
ttggatagta aatgaagagc tttgggttat atccagtaga tgcagagatt tggaaccaca
2280
tgaatgtagg aatagagaca acaaggccaa gaatcccaac tagtagataa tatcgtgaat
2340
gatatttgta aagaacgttg tattaaattc aggacactta attgatgaat cacaatcaaa
2400
taatattatt ttgcagtatc tggtacataa gtatgatata aatgataagt gccataactg
2460
tgagaatata aactataatc gtaataaaaa tttgtatagc ataaaatatt taatatttac
2520
tataaattca ataaaatcaa tgctacatga ttatgtaaga tgatgtggat tttcttaaca
2580
aaatgatttt ggcttgatcg tgatgatact attaaaaaat aatgaagttt gtctaggaca
2640
tggcattggt ttatagatag atgtcctcat acgtttattg gcacttgaca tttgtttctt

2700

tttattcttg tgaattggga tatgtggatt tataaatatt tttagaagtc tggcttgtat
2760

ttctgatact aatttttatg atataatttc tttatgaatt aattgatatt aaaggctcat
2820

ttgaagtttg cggatgattc aatgcgagca atagcgtata tattgtgatt ggttggtgca
2880

ctgcacatat aggctcgcat cgaatcatcc gccgtttgct taggaaatta taagactcat
2940

tttattaatt taaaaataga atttgggtga aaggtagacg tacggtacgg ctacggtacc
3000

taaccttcga ggcttagact aagggtaaag aaaagaccag gaaaagaaaa aatctagaat
3060

cgttaaaata ttaaatgcaa ttaaatgaaa ataaaaaagg ataaataaag atccacacca
3120

cacctatcaa ttgtggagtc taattgtttg aacttgatac aatacaatga tatctgaagt
3180

atcgtatcga aatacacacg ttatgttttt cctatttggc acatcatatt tattcttggt
3240

ttttcaaatc caagatacaa aaactcataa aatacctcag tcaccaaaaa taacactaac
3300

agatttcaaa catactgcat gattatttaa aattaaaacc gagaagtgag atgtccaaga
3360

attgtttgtt aaaaccctca aaactctaag gatattacct aataagtcgt tggatgtaat
3420

tcttatgatg atcacaaatc acacattcaa tagttcaacg caattaccga aagtagcagg
3480

gcgaaaatgt cattatcatg atcaaaggga ccatgaatgt aattgcgcaa tcagacgacg
3540

tgtcggtgaa accaaaaaat ttagaagaaa atatagctca ctcaccacca ctactcgaac
3600

ctaacaccgc attaaatatc ctacaccact tcctttctat aaagaacaca tcaacccaca

3660

aagcttttat ctcgtttaca tcgaaaccgc tatatcatta catttacaac agcgctaaaa
3720

aaatatatat aaagc
3735

<210> 19
<211> 28
<212> DNA
<213> Artificial Sequence

<220>
<223> primer TFNS1pBamHIRV

<400> 19
ggatccgctt tatatatatt tttttagc
28

<210> 20
<211> 1618
<212> DNA
<213> Torenia Fournieri

<220>
<221> misc_feature
<223> Trenia F3'5'H trancriptional region in pSPB3791

<400> 20
aatatttaaa gcacaatcta aaacgaagca cagattcatt tgtatgaagc acagattcat
60

caggggtatt ttagacaaaa acccgacgaa tatcatccgt tagatttatt ttgagcaaat
120

ccaatggccc attacttttg tccgcacata cggagaaaag taccgtccgc atttgaacca
180

gaggggatgg gagggactgc gaacataata taattcgggt caatggcgta ccaaaaaatg
240

gtcaaataaa taatgctaca agttaaatgc acgtgattct cttgttgacc gttaaatgtt
300

gatttctatt ttgatcgaat tttatagata ttagctcagc tgctgagact gagagagaga
360

ttattttaga aacggatctg atcaagatcg ctcgtgatta ttctcatcat ctcgtgtagc
420

atttagattt ggtaaaaccg taaaaatatc ttgtcttaac ttcttcctat taattccaca
480

atctcacgca aaaatgtatt tttacagcga catgccttga ggaggaattt cagtgctaaa
540

gacaaaatag acttagaaat gtgccgtttg attaattttg gtagttaaaa aaaaaaaaat
600

tatttattta tttatgctta gccataataa ttaggacgga aacaagaaga atttgcccat
660

ttttaaggcc tacgaatagt atacgggcca taactttatg ggggacattt tcaggcctac
720

ccaatatgat tgaatgtgcc tccactccaa ttatcaaaaa gaaacgccga ctgagcttga
780

gcacgatttt caaatgtcta ctccacagtg caaaaattgc acgatcaatt ctgatgaaat
840

gtattcccat ctatgtagat gaatgggagc gtttgggatc caacactcgt tttttctacc
900

tatttcgacg gttttctctg gtcaggtggt ttccgagagg ggactgaaat atacagtaca
960

gttgttgaga tattgatgag atcaatcaag tcgtgtaagc ttatctttct gaaaatataa
1020

ttatatttaa ttgattatcc taccgcttgt caattttttc tattgcgagt gtgttttggt
1080

gtaaagagta gctatcgatg ctgaagagat ttttatcaag taagatgatg tggagaacta
1140

actgattgtt tgttttgttg ccagcgatat attgatgatg atgttctttg caattagagt
1200

tattctactt aacaaacttt ttctcttgtc ataacataca catagatact gcatgctttt
1260

ccatgattca ttctatctaa taaaggtcaa tctactcccc actttcatcc atgccaatga
1320

aacacataca taaatggaaa catatcataa tttaggtgtg gccctattag ttttcttctc
1380

gttgaacgtc ggttgatggc ctaaaaaaac gtgacaaacc acacagttgt cacccaacgt
1440

actccattga tacacatata aagctcagtg attgattatt cataaacact caactggttg
1500

agcactattt gcctcttctt tgctagattc acatcgagta agtactccgt ccgacctttt
1560

attgtatggt ataatcgtaa gaaaacaaat taagcagcta gtcaccattc agtctaga
1618

<210> 21
<211> 1416
<212> DNA
<213> Torenia Fournieri

<220>
<221> misc_feature
<223> Trenia FNS transcriptional region in pSPB3789

<400> 21
ctagtagata atatcgtgaa tgatatttgt aaagaacgtt gtattaaatt caggacactt
60

aattgatgaa tcacaatcaa ataatattat tttgcagtat ctggtacata agtatgatat
120

aaatgataag tgccataact gtgagaatat aaactataat cgtaataaaa atttgtatag
180

cataaaatat ttaatattta ctataaattc aataaaatca atgctacatg attatgtaag
240

atgatgtgga ttttcttaac aaaatgattt tggcttgatc gtgatgatac tattaaaaaa
300

taatgaagtt tgtctaggac atggcattgg tttatagata gatgtcctca tacgtttatt
360

ggcacttgac atttgtttct ttttattctt gtgaattggg atatgtggat ttataaatat
420

ttttagaagt ctggcttgta tttctgatac taatttttat gatataattt ctttatgaat
480

taattgatat taaaggctca tttgaagttt gcggatgatt caatgcgagc aatagcgtat
540

atattgtgat tggttggtgc actgcacata taggctcgca tcgaatcatc cgccgtttgc
600

ttaggaaatt ataagactca ttttattaat ttaaaaatag aatttgggtg aaaggtagac
660

gtacggtacg gctacggtac ctaaccttcg aggcttagac taagggtaaa gaaaagacca
720

ggaaaagaaa aaatctagaa tcgttaaaat attaaatgca attaaatgaa aataaaaaag
780

gataaataaa gatccacacc acacctatca attgtggagt ctaattgttt gaacttgata
840

caatacaatg atatctgaag tatcgtatcg aaatacacac gttatgtttt tcctatttgg
900

cacatcatat ttattcttgg tttttcaaat ccaagataca aaaactcata aaatacctca
960

gtcaccaaaa ataacactaa cagatttcaa acatactgca tgattattta aaattaaaac
1020

cgagaagtga gatgtccaag aattgtttgt taaaaccctc aaaactctaa ggatattacc
1080

taataagtcg ttggatgtaa ttcttatgat gatcacaaat cacacattca atagttcaac
1140

gcaattaccg aaagtagcag ggcgaaaatg tcattatcat gatcaaaggg accatgaatg
1200

taattgcgca atcagacgac gtgtcggtga aaccaaaaaa tttagaagaa aatatagctc
1260

actcaccacc actactcgaa cctaacaccg cattaaatat cctacaccac ttcctttcta
1320

taaagaacac atcaacccac aaagctttta tctcgtttac atcgaaaccg ctatatcatt
1380

acatttacaa cagcgctaaa aaaatatata taaagc
1416

## Description

The present invention relates to a novel promoter. More particularly, the present invention relates to a transcription regulatory region of flavonoid 3',5'-hydroxylase (abbreviated as F3'5'H)gene or flavone synthase (abbreviated as FNS) gene derived from torenia, and to the use thereof.

### BACKGROUND ART

New traits can be imparted to plants by expressing a useful gene in a target plant using genetic recombination technology. Transgenic plants produced in this manner are already grown commercially on a wide scale. Since regulation of gene expression is primarily controlled at the transcription level, transcriptional regulation is of the greatest importance in terms of regulating gene expression. Namely, the transcription of a target gene at a suitable time, in a suitable tissue, and at a suitable strength is important for producing an industrially useful transgenic plant. In many cases, the start of transcription is controlled by a DNA sequence on the 5'-side of a translation region. A region of DNA that determines the starting site of the transcription of a gene and directly regulates the frequency thereof is referred to as a promoter. Promoters are present at a location several bp from the 5'-side of a start codon, and frequently include a sequence such as a TATA box. Cis-regulatory elements that bind various transcription regulatory factors are further present on the 5'-side, and the presence of these elements controls such factors as the timing of transcription, the tissue where transcription takes place or the strength of transcription. Transcription regulatory factors are classified into numerous families according to their amino acid sequences. Examples of some more well-known families include Myb-type transcription factors and bHLH (basic helix-loop-helix)-type transcription factors. In actuality, transcription control regions and promoters are frequently used in the same context and are not rigidly distinguished.

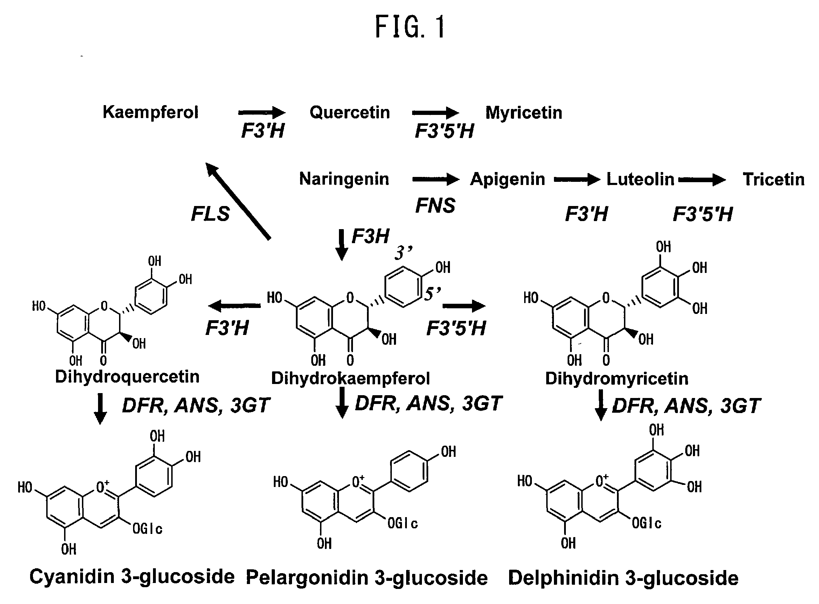

Anthocyanins, which are a main component of flower color, are one of the members of secondary metabolites generally referred to as flavonoids. The color of anthocyanins is dependent upon the structure thereof. Namely, flower color becomes blue with an increase in the number of hydroxyl groups on the B ring of anthocyanidins, which are chromophores of anthocyanins. Typical examples of anthocyanidins include delphinidin, cyanidin and pelargonidin, and the number of hydroxyl groups on the B ring of these compounds is 3, 2 and 1, respectively. Among these, delphinidin results in the bluest color (see FIG. 1). In addition, as the number of aromatic acyl groups (such as coumaroyl groups or caffeoyl groups) that modify an anthocyanin increases, the color produced by the anthocyanin is known to become blue (namely, the absorption maximum shifts towards longer wavelengths) and the stability of the anthocyanin is known to increase. Alternatively, in the case a flavonoid such as a flavone is present with an anthocyanin, the resulting color is known to become bluer and deeper. Such effects are referred to as copigment effects, and flavone is a type of copigment (see Non-Patent Document 1).

Extensive research has been conducted on enzymes involved in anthocyanin synthesis and the gene encoding those enzymes (see Non-Patent Document 1). For example, flavonoid 3'-hydroxylase (abbreviated as F3'H) and F3'5'H are enzymes that catalyze a reaction that increases the number of hydroxyl groups on the B ring. F3'H is required for synthesis of cyanidin while F3'5'H is required for the synthesis of delphinidin. Since delphinidin is not synthesized in such plants as roses, carnations and chrysanthemums, there are no blue varieties of these plants. There are examples of changing the flower color of these plants to blue by expressing F3'5'H gene in these plants (see International Publication No. WO 1996/036716, Patent Document 4 and International Publication No. WO 2009/062253). Flavones are synthesized from flavonoids by catalysis by the enzyme FNS. There are examples of changing the flower color of petunia by expressing flavone synthase gene therein (see Plant Biotechnol., 21, 377-386 (2004)).

Moreover, findings have also been obtained regarding the transcriptional regulation of genes involved in the biosynthesis of anthocyanins. A cis-regulatory element is present in the transcription control region of these genes located on the 5'-side of a start codon that binds with an Myb-type transcription regulatory factor and bHLH-type transcription regulatory factor. Myb-type transcription regulatory factor and bHLH-type transcription regulatory factor are known to control the synthesis of anthocyanins in plants such as petunia, corn and perilla (see Non-Patent Document 1).

In plants, promoters responsible for gene transcription (to also be referred to as transcription regulatory regions) consist of so-called constitutional promoters that function in any tissue and at any time in the developmental stage, organ/tissue-specific promoters that function only in specific organs and tissues, and time-specific promoters that are expressed only at specific times in the developmental stage. Constitutional promoters are frequently used as promoters for expressing useful genes in transgenic plants. Typical examples of constitutional promoters include cauliflower 35S promoter and promoters constructed on the basis thereof (see Non-Patent Document 3) and Mac1 promoter (see Non-Patent Document 4). In plants, however, many genes are expressed organ/tissue-specifically or time-specifically. This indicates that it is necessary for plants that these genes be expressed in specific tissues or organs or at specific times. There are examples of carrying out gene recombination in plants using such tissue/organ-specific or time-specific transcription regulatory regions. For example, protein has been made to accumulate in seeds by using a seed-specific transcription regulatory region.

There are several reports on promoters that function specifically in flower petals or primarily function in flower petals along with their use. For example, there are examples of changing flower color by expressing F3'5'H gene in petunia or carnation using the promoter of chalcone synthase gene derived from snapdragon or petunia. The promoter of rose chalcone synthase gene has been shown to function in rose and chrysanthemum. There are also examples of changing flower color by expressing the promoter of cineraria F3'5'H gene in petunia. In addition, there are also examples of using the flavanone 3-hydroxylase gene promoter of chrysanthemum to express F3'5'H gene in chrysanthemum petals.

However, it is difficult to predict the degree to which a promoter will function and bring about a desired phenotype in a target recombinant plant. In addition, gene silencing may occur if a base sequence identical or similar to a host plant is introduced or if numerous copies of an introduced gene are inserted or that gene is inserted repeatedly (see Non-Patent Document 5). Thus, the repeated use of the same promoter to express a plurality of exogenous genes should be avoided since it can cause gene silencing. In addition, even though the enzyme genes involved in flavonoid synthesis may function in flower petals, the timing of their expression differs. In general, flavones and flavonols are known to be expressed when flowers are younger in comparison with anthocyanins. For example, the acquisition of a plurality of promoters having different expression timing is industrially important for changing flower color.

### Prior Art Documents

### Patent Documents

Patent Document 1: International Publication No. WO96/25500
Patent Document 2: International Publication No. WO01/72984
Patent Document 3: International Publication No. WO94/28140
Patent Document 4: International Publication No. WO05/17147

### Non-Patent Documents

Non-Patent Document 1: Plant J., 54, 737-749, 2008
Non-Patent Document 2: Agricultural and Biological Chemistry, 53, 797-800, 1989
Non-Patent Document 3: Plant Cell Physiology, 1996, 37, 49-59
Non-Patent Document 4: Plant Molecular Biology, 1990, 15, 373-381
Non-Patent Document 5: Annals of Botany, 1997, 79, 3-12

### DISCLOSURE OF THE INVENTION

### Problems to be Solved by the Invention

An object of the present invention is to provide a novel promoter that is useful for changing the flower color of a plant.

### Means for Solving the Problems

As a result of conducting extensive studies to solve the aforementioned problems, the inventors of the present invention discovered that the transcription regulatory regions of torenia-derived F3'5'H gene an FNS gene are novel promoters that are useful for changing the flower color of a plant and confirmed the usefulness thereof, thereby leading to completion of the present invention.

Namely, the present invention is as described below.
[1] A nucleic acid selected from the group consisting of the following:
   (1) a nucleic acid composed of the base sequence shown in SEQ ID NO: 20;
   (2) a nucleic acid able to function as a transcription regulatory region of torenia F3'5'H gene and composed of a base sequence that has been modified by addition, deletion and/or substitution of one or several base sequences in the base sequence shown in SEQ ID NO: 20;
   (3) a nucleic acid able to function as a transcription regulatory region of torenia F3'5'H gene and hybridize under highly stringent conditions with a nucleic acid composed of a base sequence complementary to the base sequence shown in SEQ ID NO: 20; and,
   (4) a nucleic acid able to function as a transcription regulatory region of torenia F3'5'H gene and having at least 90% sequence identity with the base sequence shown in SEQ ID NO: 20.
[2] An expression vector containing the nucleic acid described in [1] above.
[3] The expression vector described in [2] above, containing the base sequence shown in SEQ ID NO: 20.
[4] A non-human host transformed by the expression vector described in [2] or [3] above.
[5] A plant, or a progeny, a portion or a tissue thereof, introduced with the nucleic acid described in [1] above.
[6] The plant, or progeny, portion or tissue thereof, described in [5] above, which is a cut flower.
[7] A processed cut flower obtained by using the cut flower described in [6] above as a raw material.
[8] A nucleic acid selected from the group consisting of the following:
   (1) a nucleic acid composed of the base sequence shown in SEQ ID NO: 12;
   (2) a nucleic acid able to function as a transcription regulatory region of torenia F3'5'H gene and composed of a base sequence that has been modified by addition, deletion and/or substitution of one or several base sequences in the base sequence shown in SEQ ID NO: 12;
   (3) a nucleic acid able to function as a transcription regulatory region of torenia F3'5'H gene and hybridize under highly stringent conditions with a nucleic acid composed of a base sequence complementary to the base sequence shown in SEQ ID NO: 12; and,
   (4) a nucleic acid able to function as a transcription regulatory region of torenia F3'5'H gene and having at least 90% sequence identity with the base sequence shown in SEQ ID NO: 12.
[9] An expression vector containing the nucleic acid described in [8] above.
[10] The expression vector described in [9] above, containing the base sequence shown in SEQ ID NO: 12.
[11] A non-human host transformed by the expression vector described in [9] or [10] above.
[12] A plant, or a progeny, a portion or a tissue thereof, introduced with the nucleic acid described in [8] above.
[13] The plant, or progeny, portion or tissue thereof, described in [12] above, which is a cut flower.
[14] A processed cut flower obtained by using the cut flower described in [13] above as a raw material.
[15] A nucleic acid selected from the group consisting of the following:
   (1) a nucleic acid composed of the base sequence shown in SEQ ID NO: 21;
   (2) a nucleic acid able to function as a transcription regulatory region of torenia FNS gene and composed of a base sequence that has been modified by addition, deletion and/or substitution of one or several base sequences in the base sequence shown in SEQ ID NO: 21;
   (3) a nucleic acid able to function as a transcription regulatory region of torenia FNS gene and hybridize under highly stringent conditions with a nucleic acid composed of a base sequence complementary to the base sequence shown in SEQ ID NO: 21; and,
   (4) a nucleic acid able to function as a transcription regulatory region of torenia FNS gene and having at least 90% sequence identity with the base sequence shown in SEQ ID NO: 21.
[16] An expression vector containing the nucleic acid described in [15] above.
[17] The expression vector described in [16] above, containing the base sequence shown in SEQ ID NO: 21.
[18] A non-human host transformed by the expression vector described in [16] or [17] above.
[19] A plant, or a progeny, a portion or a tissue thereof, introduced with the nucleic acid described in [15] above.
[20] The plant, or progeny, portion or tissue thereof, described in [19] above, which is a cut flower.
[21] A processed cut flower obtained by using the cut flower described in [20] above as a raw material.
[22] An expression vector containing the nucleic acid described in [1] above and the nucleic acid described in [15] above.
[23] The expression vector described in [22] above, containing the base sequence shown in SEQ ID NO: 20 and the base sequence shown in SEQ ID NO: 21.
[24] A non-human host transformed by the expression vector described in [22] or [23] above.
[25] A plant, or a progeny, a portion or a tissue thereof, introduced with the nucleic acid described in [1] above and the nucleic acid described in [15] above.
[26] The plant, or progeny, portion or tissue thereof, described in [25] above, which is a cut flower.
[27] An expression vector containing the nucleic acid described in [8] above and the nucleic acid described in [15] above.
[28] The expression vector described in [27] above, containing the base sequence shown in SEQ ID NO: 12 and the base sequence shown in SEQ ID NO: 21.
[29] A non-human host transformed by the expression vector described in [27] or [28] above.
[30] A plant, or a progeny, a portion or a tissue thereof, introduced with the nucleic acid described in [8] above or the nucleic acid described in [15] above.
[31] The plant, or progeny, portion or tissue thereof, described in [30] above, which is a cut flower.
[32] A processed cut flower obtained by using the cut flower described in [31] above as a raw material.

### Effects of the Invention

The use of promoter regions thought to govern the transcription of enzyme genes in torenia flower petals, namely regions that control the transcription of torenia F3'5'H gene and FNS gene, makes it possible to specifically induce transcription of an exogenous gene in tissue such as flower tissue in which flavonoids and anthocyanins accumulate. Examples of transcribed exogenous genes include, but are not limited to, genes involved in flower color, scent and the like.

### BRIEF DESCRIPTION OF THE DRAWINGS

FIG. 1 is a schematic diagram of the biosynthesis pathway of flavonoids.
FIG. 2 is a schematic diagram of a binary vector pSPB3797 for gene insertion.
FIG. 3 is a schematic diagram of a binary vector pSPB3798 for gene insertion.

### BEST MODE FOR CARRYING OUT THE INVENTION

Examples of transcription regulatory regions of the present invention include nucleic acids composed of the base sequences shown in SEQ ID NO: 20, SEQ ID NO: 12 and SEQ ID NO: 21. However, promoters composed of base sequences that have been modified by addition, deletion and/or substitution of one or more (1, 2, 3, 4, 5, 6, 7, 8, 9 or 10) bases in the base sequence shown in SEQ ID NO: 20, SEQ ID NO: 12 or SEQ ID NO: 21 are also thought to maintain activity that is the same as that of the original promoter. Thus, the present invention also relates to a nucleic acid composed of a base sequence that has been modified by addition, deletion and/or substitution of one or several base sequences in the base sequence shown in SEQ ID NO: 20, SEQ ID NO: 12 or SEQ ID NO: 21 provided the nucleic acid functions as a transcription regulatory region in flower petals.

The present invention also relates to a nucleic acid that is able to function as a transcription regulatory region of torenia F3'5'H gene or FNS gene and hybridize under highly stringent conditions with the base sequence shown in SEQ ID NO: 20, SEQ ID NO: 12 or SEQ ID NO: 21, as well as a nucleic acid that is able to function as a transcription regulatory region of torenia F3'5'H gene or FNS gene and has at least 90% sequence identity with the base sequence shown in SEQ ID NO: 20, SEQ ID NO: 12 or SEQ ID NO: 21.

Examples of these nucleic acids include nucleic acids composed of a base sequence capable of hybridizing under stringent conditions with a polynucleotide complementary to the base sequence shown in SEQ ID NO: 20, SEQ ID NO: 12 or SEQ ID NO: 21 and having sequence identity with the base sequence shown in SEQ ID NO: 20, SEQ ID NO: 12 or SEQ ID NO: 21 of preferably about 70% or more, more preferably about 80%, 81%, 82%, 83%, 84%, 85%, 86%, 87%, 88%, 89%, 90%, 91%, 92%, 93%, 94%, 95%, 96%, 97% or 98%, and most preferably about 99%.

Here, the term "stringent conditions" refers to hybridization conditions easily determined by a person with ordinary skill in the art, and are typically experimental conditions dependent upon probe length, washing temperature and salt concentration. In general, the temperature for proper annealing becomes higher the longer the probe, while the temperature becomes lower the shorter the probe. Hybridization is generally dependent on the ability of a complementary strand to reanneal denatured DNA in the case of being present in an environment at a temperature close to but lower than the melting point thereof. More specifically, an example of lowly stringent conditions consists of washing in a 5×SSC and 0.1% SDS solution under temperature conditions of 37°C to 42°C in the stage of washing the filter following hybridization. In addition, an example of highly stringent conditions consists of washing in a 0.1×SSC and 0.1% SDS solution at 65°C. Increasing the stringency of these conditions allows the obtaining of a polynucleotide having higher sequence homology or sequence identity.

The present invention also relates to a vector containing the transcription regulatory region of torenia F3'5'H gene and/or FNS gene, and a non-human host transformed by that vector.

Moreover, the present invention also relates to a plant, or a progeny, portion or tissue thereof, having a useful trait such as change of color obtained by linking the transcription regulatory region of torenia F3'5'H gene and/or FNS gene to a useful exogenous gene and introducing that exogenous gene therein, and the portion may be a cut flower. Examples of plants able to be transformed include, but are not limited to, rose, chrysanthemum, carnation, snapdragon, cyclamen, orchid, dahlia, bluebell, freesia, Transvaal daisy, gladiola, baby's breath, kalanchoe, lily, pelargonium, geranium, petunia, torenia, tulip, rice, morning glory, barley, wheat, rape, potato, tomato, poplar, banana, eucalyptus, sweet potato, soybean, alfalfa, lupine and corn.

The present invention also relates to a processed product using the aforementioned cut flower (processed cut flower). Here, examples of processed cut flowers include, but are not limited to, pressed flowers, preserved flowers, dry flowers and resin-sealed flowers using these cut flowers.

### Examples

The following provides a detailed explanation of the invention according to examples thereof.

Molecular biological techniques were in accordance with Molecular Cloning (Sambrook and Russell, 2001) unless specifically indicated otherwise. Although methods used to transform Agrobacterium, petunia and rose are described in International Publication No. WO2004/020637 or Patent Document 4, the methods are not limited thereto.

### [Example 1: Cloning of Transcription Control Region of Torenia F3'5'H Gene TBG10]

The base sequence of torenia F3'5'H cDNA is known (Molecular Breeding 6, 239-246 (2000), Gene Bank DNA Accession No. AB012925). A chromosomal DNA library of torenia was prepared using λDASHII (Agilent Technologies, Inc.) for the vector in accordance with the method recommended by the manufacturer. Torenia chromosomal DNA was prepared from the leaves of the torenia variety Summer Wave Blue (Suntory Flowers Ltd.). The resulting torenia chromosomal DNA library was screened using labeled torenia F3'5'H cDNA and the plaque of a phage that hybridized with torenia F3'5'H cDNA was recovered. Using this phage as a template, PCR was carried out using two types of oligonucleotides (T3pro: 5'-AATTAACCCTCACTAAAGGG-3' (SEQ ID NO: 1), T7pro: 5'-TAATACGACTCACTATAGGG-3' (SEQ ID NO: 2)) as primers to amplify a DNA fragment containing the sequence derived from torenia chromosomal DNA. Using this DNA as a template, PCR was carried out using one set of oligonucleotides (T3pro, THF2RV: 5'-CTATGGAAGATAACAATG-3' (SEQ ID NO: 3)) or one set of oligonucleotides (T7pro, THF2RV) as primers. An approximately 5.6 kb fragment (SEQ ID NO: 4) able to be amplified by PCR using T3pro and THF2RV was cloned in pCR2.1 TOPO (Invitrogen Corp.). The resulting plasmid was designated as pSPB3745.

Using the aforementioned phage as a template, PCR was carried out using one set of oligonucleotides (TBG10proFW: 5'-TGAAATATAAATATGAATGGG-3' (SEQ ID NO: 5), TBG10proRV: 5'-ACTGAATGGTGACTAGCTGC-3' (SEQ ID NO: 6)) as primers. The resulting DNA fragment was cloned in BluntII-TOPO (Invitrogen Corp.) to obtain plasmid pSPB3764 (containing SEQ ID NO: 7). This DNA sequence is shown in SEQ ID NO: 7. Moreover, using pSPB3764 as a template, PCR was carried out using one set of oligonucleotides (TBG10proFW, TBG10proXbaIRV: 5'-TCTAGACTGAATGGTGACTAGC-3' (SEQ ID NO: 8)) as primers. This DNA fragment was cloned in BluntII-TOPO to obtain plasmid pSPB3770. This plasmid contains in an approximately 4kb 5'-untranslated region of torenia F3'5'H and has a restrictase XbaI recognition sequence on the 3'-terminal thereof.

### [Example 2: Cloning of Transcription Control Region of Torenia F3'5'H Gene TBG16]

Plaque of the phage that hybridized with torenia F3'5'H cDNA obtained in Example 1 was allowed to proliferate to obtain phage DNA. Using this as a template, PCR was carried out using one set of oligonucleotides (T3pro, THF2RV) or one set of oligonucleotides (T7pro, THF2RV) as primers. An approximately 2.3 kb DNA fragment obtained from the (T3pro, THF2RV)-derived PCR product was cloned in pCR2.1 TOPO to obtain plasmid pSPB3746. The sequence thereof is shown in SEQ ID NO: 9. Using this plasmid as a template, PCR was carried out using a set of oligonucleotides (TBG16proFW: 5'-TCCTATTGCACTCGTTTTTTC-3' (SEQ ID NO: 10), TBG16proRV: 5'-ACTGAATGGTGACTAGCCGC^3' (SEQ ID NO: 11)) as primers. The resulting DNA fragment was cloned in BluntII-TOPO (Invitrogen Corp.) to obtain plasmid pSPB3758. The DNA sequence contained in this plasmid is shown in SEQ ID NO: 12. Moreover, using pSPB3758 as a template, PCR was carried out using one set of oligonucleotides (TBG16proFW, TBG16proBamHI: 5'-GGATCCACTGAATGGTGACTAGCC-3' (SEQ ID NO: 13)) as primers. This DNA fragment was cloned in BluntII-TOPO to obtain plasmid pSPB3768. This plasmid contains in an approximately 0.7 kb 5'-untranslated region of torenia F3'5'H and has a restrictase BamHI recognition sequence on the 3'-terminal thereof.

### [Example 3: Cloning of Transcription Control Region of Torenia FNS Gene]

The torenia chromosomal DNA library obtained in Example 1 was screened using labeled torenia FNS cDNA, and plaque of a phage hybridized with torenia FNS cDNA was recovered. The phage plaque was allowed to proliferate to prepare phage DNA. Using this DNA as a template, PCR was carried out using one set of oligonucleotides (T3pro, TFNSR3: 5'-ATTCCTAATGGGCTGAAAGTG-3' (SEQ ID NO: 14)) or one set of oligonucleotides (T7pro, TFNSR3) as primers. An approximately 4.2 kb DNA fragment (SEQ ID NO: 15) able to be amplified by PCR using T7pro and TFNSR3 was cloned in pCR2.1 TOPO. The resulting plasmid was designated as pSPB3747.

Using the aforementioned phage DNA as a template, PCR was carried out using one set of oligonucleotides (TFNS1proFW: 5'-CAAATGAAACCCCATCAGTGTC-3' (SEQ ID NO: 16), TFNS1proRV: 5'-GCTTTATATATATTTTTTTAGCGC-3' (SEQ ID NO: 17)) as primers. The amplified DNA fragment was cloned in BluntII-TOPO to obtain plasmid PSPB3759. The sequence inserted with this plasmid is shown in SEQ ID NO: 18. Using pSPB3759 as a template, PCR was carried out using one set of oligonucleotides (TNFS1proFW, TFNS1pBamHIRV: 5'-GGATCCGCTTTATATATATTTTTTTAGC-3' (SEQ ID NO: 19) as primers. This DNA fragment was cloned in BluntTOPO to obtain plasmid pSPB3769. This plasmid contains an approximately 3.6 kb 5'-untranslated region of torenia FNS and has a restrictase BamHI recognition sequence on the 3'-terminal thereof.

### [Example 4: Production of Binary Vector Containing Torenia F3'5'H and FNS Transcription Control Regions]

The plasmid pSPB3770 obtained in Example 1 was digested and blunted with SspI and further digested with XbaI. The resulting 1.6 kb DNA fragment (SEQ ID NO: 20) was linked to a DNA fragment obtained by digesting and blunting pBinPLUS with HindIII and further digesting with XbaI to obtain plasmid pSPB3791.

A DNA fragment obtained by digesting plasmid pSPB580 (described in Patent Document 4) with BamHI and PacI (containing pansy-derived F3'H'5H BP40 cDNA and a petunia D8 terminator sequence) was linked to pBinPLUS obtained by digesting with BamHI and PacI to obtain plasmid pSPB3795. A DNA fragment obtained by digesting plasmid pSPB3791 with XbaI and PacI was linked to a DNA fragment obtained by digesting pSPB3795 with XbaI and PacI to obtain binary vector pSPB3793. A TBG10-derived transcription control region, pansy-derived F3'5'H cDNA and the petunia D8 terminator were linked in this binary vector in that order.

The plasmid pSPB3768 obtained in Example 2 was digested and blunted with EcoRI and further digested with BamHI. The resulting approximately 0.7 kb DNA fragment was linked to a DNA fragment obtained by digesting and blunting pBinPLUS with HindIII and further digesting with BamHI to obtain plasmid pSPB3790. A DNA fragment obtained by digesting this with BamHI and PacI was then linked to a DNA fragment obtained by digesting pSPB580 with BamHI and PacI to obtain binary vector pSPB3792. A TBG16-derived transcription control sequence, pansy-derived F3'5'H cDNA and the petunia D8 terminator were linked in this binary vector in that order.

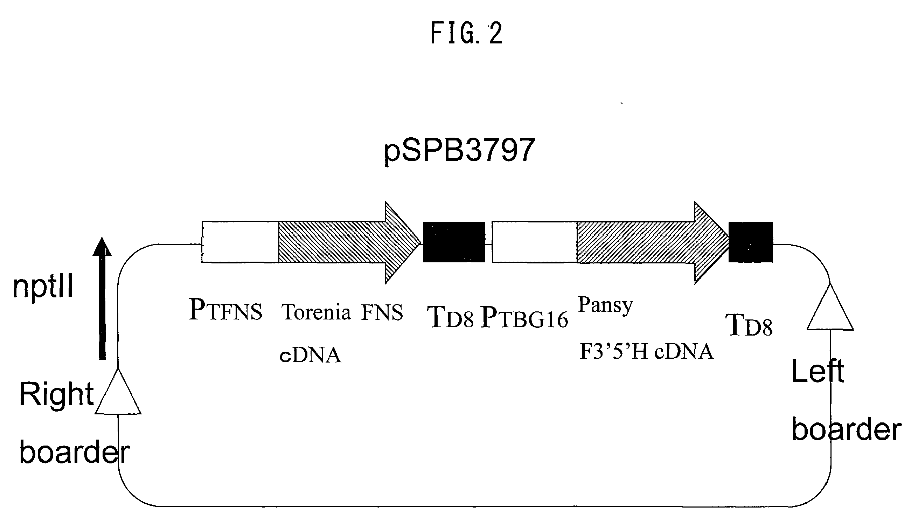

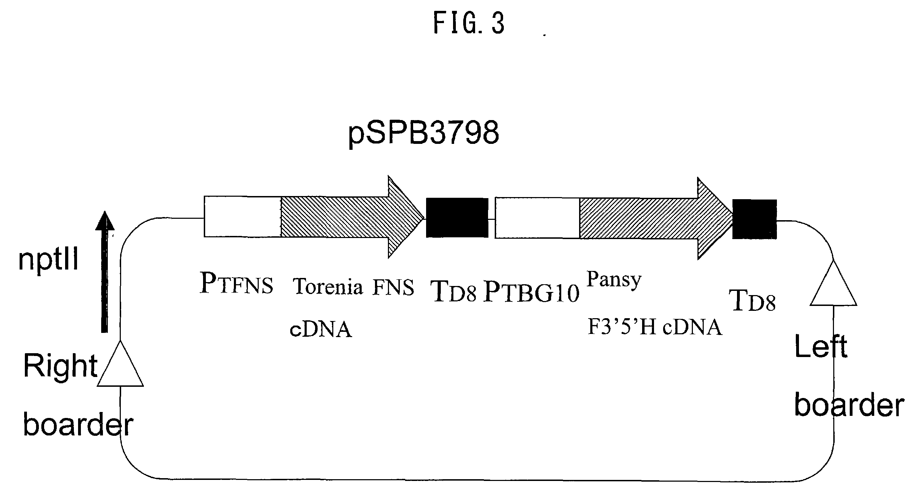

The plasmid pSPB3769 obtained in Example 3 was digested and blunted with SpeI and further digested with BamHI. The resulting approximately 1.4 kb DNA fragment (SEQ ID NO: 21) was linked to a DNA fragment obtained by digesting and blunting pSPB3383 with HindIII and further digesting with BamHI to obtain plasmid pSPB3789. A torenia FNS transcription control region, torenia FNS cDNA and petunia D8 terminator were linked in this plasmid in that order, and a DNA fragment in which they were contained was able to be recovered by cleaving with AscI. The binary vector introduced with this DNA fragment at the AscI site of pSPB3793 was designated as pSPB3798 (see FIG. 3), while the binary vector introduced with this DNA fragment at the AscI site of pSPB3792 was designated as pSPB3797 (see FIG. 2).

### [Example 5: Expression in Petunia (Results for PT315)]

Plasmid pSPB3797 or pSPB3798 described in Example 4 was introduced into Agrobacterium tumefaciens. Petunia strain Skr4xSw63 (described in International Publication No. WO93/01290) that produces light pink-colored flowers was transformed using this transformed Agrobacterium. An experimental plot derived from pSPB3797 was designated as PT314 and an experimental plot derived from pSPB3798 was designated at PT315. Two independent strains of transformants were obtained in PT314. The flower color of both strains changed to light reddish-violet. The results of analyzing the flower petals of PT314 and PT315 and the anthocyanidins of Skr4xSw63 using known methods are shown in the following Tables 1 and 2.

**[Table 1]**

| Sample | Delphinidin | Petunidin | Pelargonidin | Peonidin | Malvidin |
|---|---|---|---|---|---|
| PT314-1 | 5.4 | 98.6 | 3.8 | 20.6 | 1950 |
| PT314-2 | 4.2 | 76.5 | 3.3 | 17.6 | 1150 |
| Skr4xSw63 | 0 | 2.9 | 22.5 | 15.8 | 60.3 |

| | | | | | |
|---|---|---|---|---|---|
| Units: µg/g fresh flower petals | | | | | |

Twenty-six independent strains of transformants were obtained in PT315. Among these, the flower color of 15 strains changed to reddish-violet. The flower petals of a portion of these strains and the anthocyanidins and flavones of Skr4xSw63 were analyzed using known methods.

**[Table 2]**

| Sample | Delphinidin | Petunidin | Pelargonidin | Peonidin | Malvidin | Apigenin | Tricetin |
|---|---|---|---|---|---|---|---|
| PT315-1 | 3.0 | 72 | 0 | 2.7 | 1330 | 2.8 | 11 |
| PT315-9 | 6.8 | 126 | 0 | 0.6 | 1580 | 1.9 | 31 |
| PT315-12 | 2.0 | 33 | 1.0 | 7.0 | 393 | 44 | 9.4 |
| PT315-13 | 3.0 | 72 | 0 | 2.8 | 1550 | 4.9 | 17 |
| PT315-14 | 4.0 | 72 | 0 | 5.3 | 1290 | 5.6 | 11 |
| PT315-15 | 5.0 | 109 | 0 | 1.0 | 1630 | 4.6 | 30 |
| PT315-17 | 5.6 | 116 | 0 | 8.2 | 1690 | 18 | 27 |
| PT315-19 | 1.9 | 39 | 1.5 | 11 | 756 | 3.0 | 7.0 |
| PT315-20 | 3.6 | 93 | 0 | 4.8 | 1190 | 3.3 | 17 |
| PT315-21 | 10 | 185 | 0 | 14 | 1130 | 1.4 | 8.1 |
| PT315-25 | 5.8 | 101 | 0 | 0.6 | 1540 | 2.4 | 11 |
| PT315-26 | 3.3 | 71 | 0 | 1 | 862 | 3.5 | 25 |
| Skr4xSw63 | 0 | 3.2 | 5.6 | 11 | 74 | 0 | 0 |

| | | | | | | | |
|---|---|---|---|---|---|---|---|
| Units: µg/g fresh flower petals | | | | | | | |

The above results indicate that a TBG10-derived transcription control region contained in pSPB3770, a TBG16-derived transcription control region contained in pSPB3768, and an FNS-derived transcription control region contained in pSPB3759 demonstrate their functions in different strains of petunia as well.

### [Example 6: Introduction of pSPB3798 into Rose Variety "Ocean Song"]

Binary plasmid pSPB3798 described in Example 4 was introduced into Agrobacterium tumefaciens strain Agl0. Rose variety "Ocean Song" was transformed using this transformed Agrobacterium to obtain 15 transformants. Among the 15 transformants subjected to anthocyanidin analysis, accumulation of delphinidin was confirmed in 5 transformants. The maximum delphinidin content was 7.3% (mean: 5.0%). In addition, as a result of analyzing flavonols and flavones, myricetin was detected in 8 of 9 transformants and tricetin was detected in 7 transformants. Delphinidin, myricetin and tricetin were all formed by the action of pansy-derived F3'5'H, and the transcription control region of torenia-derived TBG10 gene was indicated to function in rose.

On the other hand, since previously absent accumulation of flavones (tricetin, luteolin, apigenin) was able to be confirmed in all the transformants subjected to analysis, the transcription control region of torenia-derived FNS gene was also indicated to function in rose. Furthermore, the total amount of flavones was a maximum of 0.6588 mg per 1 g of fresh flower petals.

The analysis values of representative transformants are shown in the following Table 3.

**[Table 3]**

| Plant No. | Del (%) | Anthocyanidins (mg/g) | | | Flavonols (mg/g) | | | Flavones (mg/g) | | | |
|---|---|---|---|---|---|---|---|---|---|---|---|
| | | Del | Cya | Pel | M | Q | K | Tri | Lut | Api | Total |
| Ocean Song Control | 0.05 | 0.0000 | 0.0324 | 0.0000 | 0.0000 | 2.6657 | 0.1730 | 0.0000 | 0.0000 | 0.0000 | 0.0000 |
| 1 | 7.3% | 0.0006 | 0.0076 | 0.0000 | | | | | | | |
| 2 | 6.0% | 0.0006 | 0.0100 | 0.0000 | 0.0662 | 1.3835 | 0.2738 | 0.0431 | 0.2928 | 0.0138 | 0.3498 |
| 3 | 5.4% | 0.0002 | 0.0036 | 0.0000 | | | | | | | |
| 4 | 4.8% | 0.0004 | 0.0090 | 0.0000 | | | | | | | |
| 5 | 1.4% | 0.0002 | 0.0141 | 0.0000 | 0.0109 | 1.7564 | 0.2911 | 0.0110 | 0.1680 | 0.0088 | 0.1878 |
| 6 | 0.0% | 0.0000 | 0.0019 | 0.0000 | 0.0041 | 0.5825 | 0.2806 | 0.0112 | 0.3098 | 0.0206 | 0.3417 |
| 7 | 0.0% | 0.0000 | 0.0026 | 0.0000 | 0.0000 | 0.05060 | 0.3112 | 0.0063 | 0.3153 | 0.0222 | 0.3439 |
| 8 | 0.0% | 0.0000 | 0.0106 | 0.0000 | 0 0075 | 1.4460 | 0.4400 | 0.0056 | 0.0825 | 0.0059 | 0.0941 |
| 9 | 0.0% | 0.0000 | 0.0037 | 0.0000 | 0.0137 | 0.8253 | 0.2856 | 0.0577 | 0.5679 | 0.332 | 0.6588 |
| 10 | 0.0% | 0.0000 | 0.0492 | 0.0000 | 0.0341 | 3.5195 | 0.4933 | 0.0000 | 0.1222 | 0.0066 | 0.1289 |

| | | | | | | | | | | | |
|---|---|---|---|---|---|---|---|---|---|---|---|
| Del: delphinidin, Cya: cyanidin, Pel: pelargonidin, M: myricetin, Q: quercetin, K: kaempferol, Tri: tricetin, Lut: luteolin, Api: apigenin Dei(%): Percentage of delphinidin among total anthocyanidins | | | | | | | | | | | |

### [Example 7: Introduction of pSPB3797 into Rose Variety "Ocean Song"]

Binary vector pSPB3797 described in Example 4 was introduced into the lavender-colored rose variety "Ocean Song" and 7 transformants were obtained. Accumulation of delphinidin was unable to be confirmed in any of the 7 transformants analyzed for anthocyanidins. In addition, as a result of analyzing for flavonols and flavones, there were no transformants in which myricetin or tricetin was detected. These results indicate that the promoter of torenia-derived TBG16 gene does not function in rose.

On the other hand, since accumulation of flavones (luteolin, apigenin) due to the action of torenia-derived FNS was able to be confirmed in all analyzed transformants, the promoter of torenia-derived FNS gene was indicated to function in rose. Furthermore, the total amount of flavones was a maximum of 0.1704 mg per 1 g of fresh flower petals.

Analysis values of representative transformants are shown in the following Table 4.

**[Table 4]**

| Plant No. | Del (%) | Anthocyanidins (mg/g) | | | Flavonols (mg/g) | | | Flavones (mg/g) | | | |
|---|---|---|---|---|---|---|---|---|---|---|---|
| | | Del | Cya | Pel | M | Q | K | Tri | Lut | Api | Total |
| Ocean Song Control | 0.05 | 0.0000 | 0.0324 | 0.0000 | 0.0000 | 2.6657 | 0.1730 | 0.0000 | 0.0000 | 0.0000 | 0.0000 |
| 1 | 0.0% | 0.0000 | 0.0123 | 0.0000 | 0.0000 | 0.1610 | 0.2288 | 0.0000 | 0.1405 | 0.0076 | 0.1481 |
| 2 | 0.0% | 0.0000 | 0.0034 | 0.0000 | 0.0000 | 0.8202 | 0.2264 | 0.0000 | 0.1367 | 0.0082 | 0.1449 |
| 3 | 0.0% | 0.0000 | 0.0106 | 0.0000 | 0.0000 | 1.2625 | 0.5670 | 0.0000 | 0.1292 | 0.0102 | 0.1394 |
| 4 | 0.0% | 0.0000 | 0.0190 | 0.0000 | 0.0000 | 1.9873 | 0.9176 | 0.0000 | 0.0702 | 0.0048 | 0.0750 |
| 5 | 0.0% | 0.0000 | 0.0077 | 0.0000 | 0.0000 | 1.6323 | 0.4398 | 0.0000 | 0.1651 | 0.0053 | 0.1704 |
| 6 | 0.0% | 0.0000 | 0.0208 | 0.0000 | 0.0000 | 2.4281 | 0.6282 | 0.0000 | 0.1067 | 0.0052 | 0.1119 |
| 7 | 0.0% | 0.0000 | 0.0154 | 0.0000 | 0.0000 | 2.1013 | 0.3322 | 0.0000 | 0.1528 | 0.0062 | 0.1590 |

| | | | | | | | | | | | |
|---|---|---|---|---|---|---|---|---|---|---|---|
| Del: delphinidin, Cya: cyanidin, Pel: pelargonidin, M: myricetin, Q: quercetin, K: kaempferol, Tri: tricetin, Lut: luteolin, Api: apigenin Del(%): Percentage of delphinidin among total anthocyanidins | | | | | | | | | | | |

### INDUSTRIAL APPLICABILITY

Promoter regions thought to govern transcription of enzyme genes in torenia flowers, namely the transcription regulatory regions of torenia flavonoid 3',5'-hydroxlyase gene and flavone synthase gene, were determined to function as transcription regulatory regions in the flower petals of different species of flowers in the form of petunia and rose. Thus, it is possible to specifically induce transcription of an exogenous gene in tissue such as flower tissue in which anthocyanins accumulate by using these transcription regulatory regions. Examples of transcribed exogenous genes include, but are not limited to, genes involved in flower color, scent and the like.

## Claims

**1.** A nucleic acid selected from the group consisting of the following:
(1) a nucleic acid composed of the base sequence shown in SEQ ID NO: 20;
(2) a nucleic acid able to function as a transcription regulatory region of torenia F3'5'H gene and composed of a base sequence that has been modified by addition, deletion and/or substitution of one or several base sequences in the base sequence shown in SEQ ID NO: 20;
(3) a nucleic acid able to function as a transcription regulatory region of torenia F3'5'H gene and hybridize under highly stringent conditions with a nucleic acid composed of a base sequence complementary to the base sequence shown in SEQ ID NO: 20; and,
(4) a nucleic acid able to function as a transcription regulatory region of torenia F3'5'H gene and having at least 90% sequence identity with the base sequence shown in SEQ ID NO: 20.

**2.** An expression vector containing the nucleic acid according to claim 1.

**3.** The expression vector according to claim 2, containing the base sequence shown in SEQ ID NO: 20.

**4.** A non-human host transformed by the expression vector according to claim 2 or 3.

**5.** A plant, or a progeny, a portion or a tissue thereof, introduced with the nucleic acid according to claim 1.

**6.** The plant, or progeny, portion or tissue thereof, according to claim 5, which is a cut flower.

**7.** A processed cut flower obtained by using the cut flower according to claim 6 as a raw material.

**8.** A nucleic acid selected from the group consisting of the following:
(1) a nucleic acid composed of the base sequence shown in SEQ ID NO: 12;
(2) a nucleic acid able to function as a transcription regulatory region of torenia F3'5'H gene and composed of a base sequence that has been modified by addition, deletion and/or substitution of one or several base sequences in the base sequence shown in SEQ ID NO: 12;
(3) a nucleic acid able to function as a transcription regulatory region of torenia F3'5'H gene and hybridize under highly stringent conditions with a nucleic acid composed of a base sequence complementary to the base sequence shown in SEQ ID NO: 12; and,
(4) a nucleic acid able to function as a transcription regulatory region of torenia F3'5'H gene and having at least 90% sequence identity with the base sequence shown in SEQ ID NO: 12.

**9.** An expression vector containing the nucleic acid according to claim 8.

**10.** The expression vector according to claim 9, containing the base sequence shown in SEQ ID NO: 12.

**11.** A non-human host transformed by the expression vector according to claim 9 or 10.

**12.** A plant, or a progeny, a portion or a tissue thereof, introduced with the nucleic acid according to claim 8.

**13.** The plant, or progeny, portion or tissue thereof, according to claim 12, which is a cut flower.

**14.** A processed cut flower obtained by using the cut flower according to claim 13 as a raw material.

**15.** A nucleic acid selected from the group consisting of the following:
(1) a nucleic acid composed of the base sequence shown in SEQ ID NO: 21;
(2) a nucleic acid able to function as a transcription regulatory region of torenia FNS gene and composed of a base sequence that has been modified by addition, deletion and/or substitution of one or several base sequences in the base sequence shown in SEQ ID NO: 21;
(3) a nucleic acid able to function as a transcription regulatory region of torenia FNS gene and hybridize under highly stringent conditions with a nucleic acid composed of a base sequence complementary to the base sequence shown in SEQ ID NO: 21; and,
(4) a nucleic acid able to function as a transcription regulatory region of torenia FNS gene and having at least 90% sequence identity with the base sequence shown in SEQ ID NO: 21.

**16.** An expression vector containing the nucleic acid according to claim 15.

**17.** The expression vector according to claim 16, containing the base sequence shown in SEQ ID NO: 21.

**18.** A non-human host transformed by the expression vector according to claim 16 or 17.

**19.** A plant, or a progeny, a portion or a tissue thereof, introduced with the nucleic acid according to claim 15.

**20.** The plant, or progeny, portion or tissue thereof, according to claim 19, which is a cut flower.

**21.** A processed cut flower obtained by using the cut flower according to claim 19 as a raw material.

**22.** An expression vector containing the nucleic acid according to claim 1 and the nucleic acid according to claim 15.

**23.** The expression vector according to claim 22, containing the base sequence shown in SEQ ID NO: 20 and the base sequence shown in SEQ ID NO: 21.

**24.** A non-human host transformed by the expression vector according to claim 22 or 23.

**25.** A plant, or a progeny, a portion or a tissue thereof, introduced with the nucleic acid according to claim 1 and the nucleic acid according to claim 15.

**26.** The plant, or progeny, portion or tissue thereof, according to claim 25, which is a cut flower.

**27.** An expression vector containing the nucleic acid according to claim 8 and the nucleic acid according to claim 15.

**28.** The expression vector according to claim 27, containing the base sequence shown in SEQ ID NO: 12 and the base sequence shown in SEQ ID NO: 21.

**29.** A non-human host transformed by the expression vector according to claim 27 or 28.

**30.** A plant, or a progeny, a portion or a tissue thereof, introduced with the nucleic acid according to claim 8 or the nucleic acid according to claim 15.

**31.** The plant, or progeny, portion or tissue thereof, according to claim 30, which is a cut flower.

**32.** A processed cut flower obtained by using the cut flower according to claim 31 as a raw material.

**1.** A nucleic acid selected from the group consisting of the following:
(1) a nucleic acid composed of the base sequence shown in SEQ ID NO: 21;
(2) a nucleic acid able to function as a transcription regulatory region of torenia flavone synthase gene and composed of a base sequence that has been modified by addition, deletion and/or substitution of one to ten bases in the base sequence shown in SEQ ID NO: 21;
(3) a nucleic acid able to function as a transcription regulatory region of torenia flavone synthase gene and hybridize under highly stringent conditions with a nucleic acid composed of a base sequence complementary to the base sequence shown in SEQ ID NO: 21; and,
(4) a nucleic acid able to function as a transcription regulatory region of torenia flavone synthase gene and having at least 90% sequence identity with the base sequence shown in SEQ ID NO: 21.

**2.** An expression vector containing the nucleic acid according to claim 1.

**3.** The expression vector according to claim 2, containing the base sequence shown in SEQ ID NO: 21.

**4.** A non-human host transformed by the expression vector according to claim 2 or 3.

**5.** A plant, or a progeny, a portion or a tissue thereof, introduced with the nucleic acid according to claim 1.

**6.** The plant, or progeny, portion or tissue thereof, according to claim 5, which is a cut flower.

**7.** A processed cut flower obtained by using the cut flower according to claim 6 as a raw material.
